# EUROPÄISCHE PATENTSCHRIFT

(11) **EP 0 356 964 B1**
(45) Veröffentlichungstag und Bekanntmachung des Hinweises auf die Patenterteilung: **18.01.1995**
(21) Anmeldenummer: 89115852.9
(22) Anmeldetag: 28.08.1989
(51) Int. Cl.: G01N 33/53, G01N 33/563

(54) **Verfahren zur Bestimmung einer spezifisch bindefähigen Substanz**
Method for the determination of a specific binding substance
Méthode pour la détermination d'une substance de liaison spécifique

(30) Priorität: 29.08.1988 DE 3829245
(43) Veröffentlichungstag der Anmeldung: 07.03.1990
(73) Patentinhaber: BOEHRINGER MANNHEIM GMBH, 68298 Mannheim (DE)
(72) Erfinder: Schenk, Roland, Dr., D-8120 Weilheim (DE); Zdunek, Dietmar, Dr., D-8000 München 40 (DE)
(74) Vertreter: Huber, Bernhard, Dipl.-Chem.

(56) Entgegenhaltungen:
- EP-A- 0 138 297
- EP-A- 0 147 848
- WO-A-88/05913
- GB-A- 2 013 211
- US-A- 4 829 011

## Beschreibung

Die Erfindung betrifft ein Verfahren zur Bestimmung einer spezifisch bindefähigen Substanz durch Inkubation der Probelösung mit mindestens zwei Rezeptoren R₁ und R₂, wobei R₁ und R₂ miteinander bindefähig und R₁ mit der zu bestimmenden Substanz bindefähig ist und Messung der bei der Reaktion auftretenden Agglutination sowie ein dazu geeignetes Reagenz.

In Körperflüssigkeiten und Geweben kommen sehr viele Substanzen vor, die mit einem spezifischen Bindungspartner bindefähig sind und als Parameter für bestimmte Erkrankungen oder den Gesundheitszustand des menschlichen Körpers dienen. Hierzu zählen unter anderem Haptene wie z. B. Hormone, Proteine wie Tumormarker, Proteinhormone und virale Proteine sowie Antikörper. Auch ist zur Überwachung einer medikamentösen Behandlung häufig die Bestimmung von Arzneistoffen im Blut erforderlich. Da diese Substanzen oft nur in sehr geringen Mengen vorkommen, verwendet man zu ihrem Nachweis Verfahren nach dem Prinzip des Immunoassays. Dazu gibt es viele Varianten. Die verschiedenen immunologischen Bestimmungsverfahren lassen sich in homogene und heterogene Verfahren einteilen. Bei den heterogenen Verfahren ist immer eine Festphasenreaktion beteiligt, um den gebundenen Anteil der markierten Komponenten von dem ungebundenen abzutrennen. Bei dieser Verfahrensart läßt sich die Markierung gut bestimmen, nachteilig ist jedoch, daß die heterogene Reaktion lange dauert.

Bei der homogenen Verfahrensvariante erfolgt keine Trennung von gebundener Markierung und ungebundener Markierung, so daß eine Differenzierung von gebundener und ungebundener Markierung durch andere Methoden erfolgen muß.

Hierzu gibt es verschiedene Möglichkeiten. So können beispielsweise konjugierte Enzyme als Markierung verwendet werden, die nur dann ihre Enzymaktivität erhalten, wenn sie an das zu bestimmende Hapten oder Antigen gebunden werden oder durch die zu bestimmende Substanz aktiviert werden. Eine weitere Möglichkeit besteht darin, als Markierung eine fluoreszierende Substanz zu verwenden, deren Fluoreszenz durch Bindung an die zu bestimmende Substanz entweder in einen anderen Wellenbereich verschoben wird oder deren Polarisation geändert wird.

Die Nachteile dieser bekannten Verfahren liegen insbesondere darin, daß die Probe häufig den Test störende Komponenten enthält, was eine Probenvorbehandlung zur Beseitigung dieser Substanzen erforderlich macht. Außerdem ist eine aufwendige Optimierung für jeden Parameter erforderlich, z. B. müssen die Enzyme parameterabhängig modifiziert werden.

Weiterhin ist aus EP-OS 79 962 ein Verfahren bekannt, bei dem die das zu bestimmende Hapten enthaltende Lösung mit haptenbeschichteten Latexpartikeln oder haptenbeschichtetem Albumin in Kontakt gebracht wird. Durch Zugabe von mit den Hapten bindefähigen Antikörpern erfolgt eine Agglutinationsreaktion. Da das an die Latexteilchen bzw. an das Albumin gebundene Hapten mit dem in der Probe enthaltenen Hapten konkurriert, ist die Agglutinationsreaktion umso geringer, je mehr Hapten in der Probe enthalten ist. Der Nachteil dieses Verfahrens liegt darin, daß für jede zu bestimmende Substanz spezielle Teilchen zur Verfügung gestellt werden müssen und jeder Parameter individuell optimiert werden muß.

Ein weiteres Verfahren zum Nachweis von Proteinen, das auf der Auswertung einer Agglutinationsreaktion beruht, ist aus DE-OS 27 49 956 bekannt. Dabei werden Antikörper gegen die zu bestimmende Substanz direkt an die agglutinierbaren Teilchen gebunden. Durch diese Bindung kann jedoch die Reaktivität der Antikörper beeinträchtigt werden. Außerdem ist ein derartiges Bestimmungsverfahren anfällig für Störungen durch Rheumafaktoren.

WO-A-8 805 913 betrifft ein Verfahren, in welchem ein Analyt bestimmt werden muß, der in einer Probe vorliegt, die ein Partikel enthält und dieses Partikel dann eine wesentliche Rolle in der Reaktion spielt. Das Teilchen kann aus roten Blutkörperchen bestehen und das Verfahren am Ganzblut, welches diese roten Blutkörperchen enthält, durchgeführt werden. Das "Konstrukt" bindet dann einerseits an die roten Blutkörperchen und andererseits an die zu bestimmende Substanz.

In EP-A-0138297 wird ein diagnostisches Teilchen offenbart, welches aus einem polymeren Träger bestimmter Größe besteht, der mit einer Vielzahl von Avidinmolekülen über Amidbindungen gebunden ist und an die Avidinmoleküle wiederum durch Komplexbildung ein Konjugat aus Biotin und einem den Analyten spezifisch erkennenden Antikörper gebunden ist.

Bei der Herstellung der diagnostischen Partikel wird freie Biotinsäure zugesetzt, um zu verhindern, daß der "exzessiv biotinylierte Antikörper" die Avidingruppen auf dem Kernteilchen komplexiert. Die Biotinsäure blockiert einen Teil der Bindungsstellen auf dem Avidin, die sonst durch den biotinylierten Antikörper besetzt würden.

Nachteil aller bekannten kompetitiven homogenen Agglutinations-Immunoassays ist es, daß die Rohstoffe sehr aufwendig parameterspezifisch optimiert werden müssen. Bei all diesen Tests bestehen einander entgegengesetzte Anforderungen für eine optimale Differenzierung und optimale Empfindlichkeit, da einerseits die Konzentration des partikelförmigen Reagenzes limitiert sein soll, damit die Konkurrenzreaktion mit der Probe sinnvoll möglich ist und andererseits das partikelförmige Reagenz hochkonzentriert und hochmarkiert sein soll, um eine genügende Signaländerung pro Zeiteinheit zu erzielen. Die Abstimmung dieser Anforderungen führt zu eingeschränkter Empfindlichkeit und Störanfälligkeit, die oft nur durch spezifische Probenvorbehandlung beseitigt werden können.

Aufgabe der vorliegenden Erfindung war es daher, ein homogenes Bestimmungsverfahren zur Verfügung zu stellen, das den Nachweis von Substanzen mit hoher Empfindlichkeit und Genauigkeit ermöglicht und die oben beschriebenen Nachteile nicht hat.

Diese Aufgabe wird gelöst durch ein Verfahren zur Bestimmung einer spezifisch bindefähigen Substanz durch Inkubation der Probelösung mit mindestens zwei Rezeptoren R₁ und R₂, wobei R₁ und R₂ miteinander bindefähig sind und R₁ mit der zu bestimmenden Substanz spezifisch bindefähig ist und Messung der bei der Reaktion auftretenden Agglutination, welches dadurch gekennzeichnet ist, daß als Rezeptor R₁ ein Konjugat aus einem Partner eines spezifisch bindenden Paares P der nur eine Bindungstelle für R₂ besitzt und einer mit der zu bestimmenden Substanz spezifisch bindefähigen Komponente K und als R₂ ein Rezeptor, der mindestens zwei Bindungsstellen für P aufweist und in der Probelösung nicht schon natürlich vorhanden ist, verwendet wird.

Das erfindungsgemäße Verfahren ist geeignet zur Bestimmung praktisch aller in Körperflüssigkeiten oder Gewebeextrakten nachzuweisenden zu einer spezifischen Bindung befähigen Substanzen, wobei niedrigkonzentrierte Substanzen ebenso gut nachweisbar sind wie hochkonzentrierte. Die Empfindlichkeit und Genauigkeit des Verfahrens ist gegenüber den bisher bekannten Verfahren verbessert. Die Erfindung bietet die Möglichkeit, mit einfachen Reagenzien schnell und zuverlässig Bestimmungen durchzuführen.

Das Verfahren ist sowohl zur Bestimmung von monovalenten spezifisch bindefähigen als auch bi- oder polyvalenten spezifisch bindefähigen Substanzen geeignet. Als monovalent wird dabei eine Substanz bezeichnet, die für einen spezifisch bindefähigen Partner nur eine Bindungsstelle besitzt. Als Beispiele sind hier zu nennen Haptene, z. B. Arzneistoffe. Als di- oder polyvalent wird eine Substanz bezeichnet, die für einen spezifisch bindefähigen Partner zwei oder mehr Bindungsstellen aufweist, wie z.B. Proteinhormone wie HCG oder TSH, Antigene und Proteine, Tumormarker wie CEA, virale Proteine und Antikörper.

Unter Epitop wird in der Beschreibung der vorliegenden Erfindung eine Bindungsstelle verstanden, die eine spezifische Bindung mit einer anderen Substanz eingehen kann. Beispiele für Epitope sind antigene Determinanten auf Antigenen und Haptenen oder aber auch spezifische Bindungsstellen auf Proteinen.

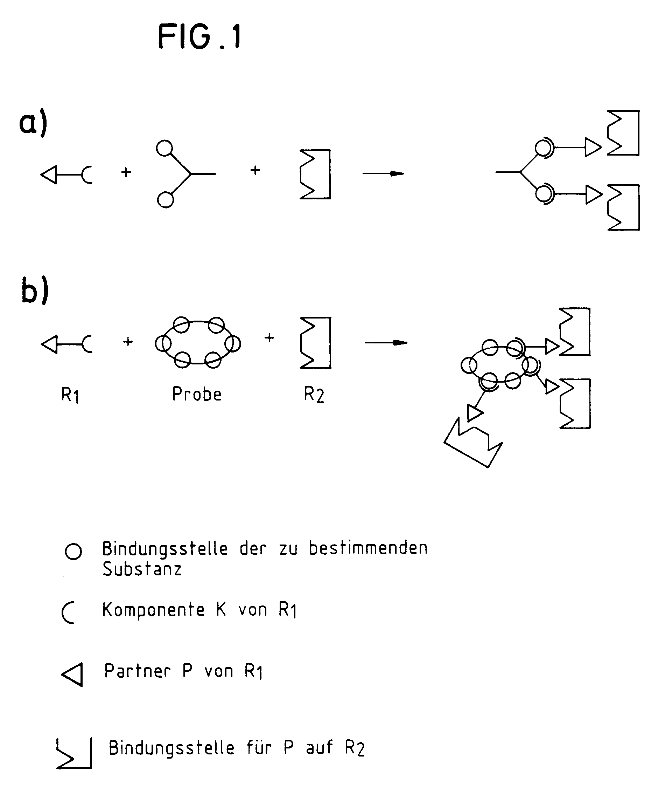

Zur Bestimmung wird die Probelösung mit mindestens zwei Rezeptoren R₁ und R₂ inkubiert. Die Rezeptoren R₁ und R₂ sind dabei miteinander bindefähig, und R₁ ist außerdem mit der zu bestimmenden Substanz spezifisch bindefähig. Mit dem erfindungsgemäßen Verfahren können verschiedene Reaktionsprinzipien durchgeführt werden. In Fig. 1 sind zwei Varianten dargestellt, die zum Nachweis einer bi- bzw. polyvalenten Substanz geeignet sind.

Bei der in Fig. 1a dargestellten Durchführungsform wird einer Probelösung Rezeptor R₁, der ein Konjugat aus einem Partner eines spezifisch miteinander bindenden Paares P und einer mit der zu bestimmenden Substanz spezifisch bindefähigen Komponente K ist, zugegeben. Rezeptor R₁ bindet dann über Komponente K an die zu bestimmende Substanz, die im dargestellten Schema ein Antikörper ist. Dabei bilden sich Komplexe, wobei an jedem Antikörper jeweils zwei Rezeptoren R₁ über K gebunden sind.

Gleichzeitig mit Rezeptor R₁ oder nach einem bestimmten Zeitraum wird Rezeptor R₂ zugegeben, der mindestens 2 und bevorzugt eine Vielzahl Bindungsstellen für P aufweist. Dabei kommt es zur Bindung des Rezeptors R₁ über P an Rezeptor R₂. Da jeder Antikörper zwei Rezeptoren R₁ und damit zwei für die Bindung an R₂ befähigte Partner P aufweist, kommt es zur Vernetzung bzw. Agglutination, die wiederum eine photometrisch detektierbare Trübung bzw. Trübungsänderung verursacht. Je mehr von dem zu bestimmenden Antikörper in der Lösung enthalten ist, desto stärker ist die Vernetzung und desto stärker ist auch die Trübungszunahme. Somit ist der Umfang der Agglutination ein direktes Maß für die zu bestimmende Substanz. Die Auswertung erfolgt dabei über eine Eichkurve.

Die Durchführungsvariante 1b) dient zum Nachweis von polyvalenten Substanzen wie z.B. Proteinen, die eine Vielzahl spezifischer Bindungsstellen aufweisen. Das Prinzip ist dabei das gleiche wie bei Variante 1a). Die zu bestimmende Substanz kann dabei jedoch mehr als zwei Rezeptoren R₁ binden, so daß nach Zugabe von Rezeptor R₂ nicht nur eine lineare Vernetzung, sondern sogar eine dreidimensionale Vernetzung erfolgen kann. Auch hier ist wieder der Umfang der Agglutination ein direktes Maß für die zu bestimmende Substanz, wobei die Auswertung wiederum über eine Eichkurve erfolgt.

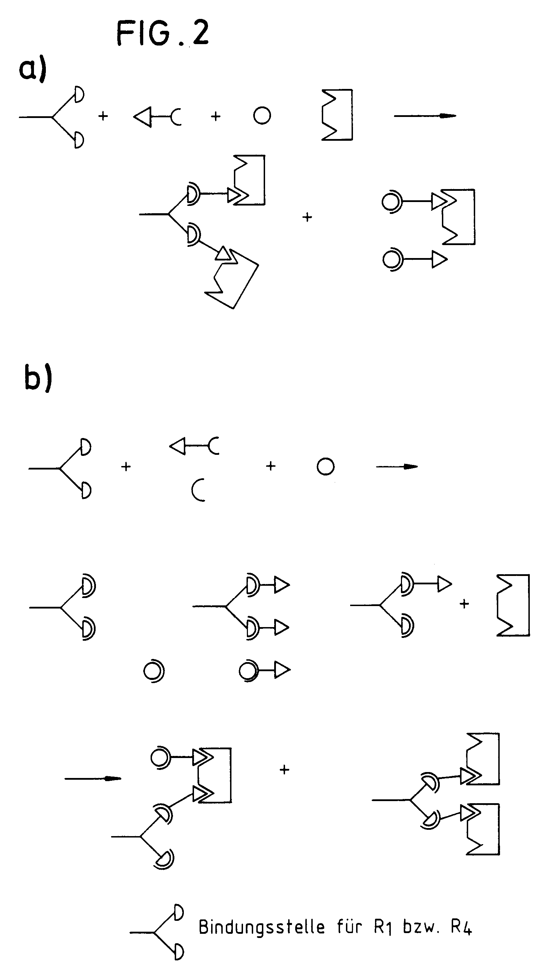

Das erfindungsgemäße Verfahren ist ebenso geeignet zur Durchführung sogenannter Uptake-Tests. Dazu werden mindestens ein weiterer Rezeptor R₃ und gegebenenfalls Rezeptor R₄ verwendet. Zwei Varianten sind in Fig. 2 dargestellt. Die bevorzugte Durchführungsform zeigt Fig. 2a. Hier wird ein Verfahren zur Bestimmung der Thyroxinbindekapazität, d.h. der Anzahl freier Bindungsstellen, die durch TBG (Thyroxin bindendes Globulin) zur Verfügung gestellt werden, bestimmt. Dazu werden der Probelösung Rezeptor R₁, der ein Konjugat aus Thyroxin und Biotin ist und Rezeptor R₃, der ein Anti-T₄-Antikörper ist, zugegeben. Dabei konkurrieren TBG in der Probelösung, das noch freie Bindungsstellen für Thyroxin hat und Rezeptor R₃ um Bindung an das Thyroxin, das im Rezeptor R₁ enthalten ist. Gleichzeitig mit den Rezeptoren R₁ und R₃ oder anschließend an die Inkubation wird Rezeptor R₂ zugegeben, der im vorliegenden Fall Streptavidin ist. Alle Komplexe, die sich aus TBG bzw. Rezeptor R₃ mit Rezeptor R₁ gebildet haben, können mit Streptavidin binden. Eine Vernetzung erfolgt jedoch nur durch Komplexe, bei denen an Rezeptor R₃ zwei Rezeptoren R₁ gebunden sind. Je mehr TBG mit freien Bindungsstellen in der Probelösung nun vorhanden ist, desto mehr Rezeptor R₁ bindet an das TBG und desto geringer ist der Umfang der Vernetzung bzw. der Agglutination und die Trübungszunahme. Somit ist die Trübungszunahme ein indirektes Maß für den Gehalt an freien Bindungsstellen für Thyroxin. Die Auswertung kann über eine Eichkurve erfolgen.

Eine Variante des Uptake-Testes ist in Fig. 2b dargestellt. Hier wird zusätzlich zu den drei im Beispiel 2a verwendeten Rezeptoren noch ein vierter Rezeptor R₄ verwendet, der Thyroxin ist. Nach Zugabe der Rezeptoren R₁, R₃ und R₄ konkurrieren TBG mit freien Bindungsstellen und Rezeptor R₃ um die Bindung an die Rezeptoren R₁ und R₄. Nur die Komplexe, bei denen Rezeptor R₁ gebunden ist, können nach Zugabe des Rezeptors R₂ an R₂ binden. Nur die Komplexe aus Rezeptor R₃ und zwei Rezeptoren R₁ können nach Zugabe von Rezeptor R₂ zu einer Vernetzung und somit einer Trübungszunahme führen. Je mehr TBG mit freien Bindungsstellen nun in der Probelösung vorhanden sind, desto mehr Rezeptor R₁ wird an TBG gebunden. Diese Komplexe aus TBG und Rezeptor R₁ können keine Vernetzung herbeiführen und vermindern daher den Umfang der Vernetzung und somit die Trübungszunahme. Auch hier ist somit die Trübungszunahme ein indirektes Maß für das in der Probelösung vorhandene TBG mit freien Bindungsstellen.

Für das erfindungsgemäß definierte Verfahrensprinzip gibt es also viele Durchführungsvarianten. In jedem Fall sind mindestens zwei Rezeptoren erforderlich. Die zu bestimmende Substanz kann jede zu einer spezifischen Bindung fähige Substanz sein und insbesondere, wie oben definiert, ein bivalentes oder polyvalentes Antigen, Antikörper oder Protein sein.

Als erster Rezeptor R₁ wird ein Konjugat verwendet, das aus einem Partner eines spezifisch bindenden Paares P und einer mit der zu bestimmenden Substanz spezifisch bindefähigen Komponente K besteht. Spezifisch miteinander bindende Paare sind an sich bekannt. Geeignete Bindungspaare (P-R₂) sind insbesondere Biotin-Streptavidin bzw. Avidin; Hapten-Antikörper; Antigen-Antikörper; Concavalin-Antikörper; Zucker-Lectin; Hapten-Bindeprotein, z.B. Thyroxin bindendes Globulin und Thyroxin oder Oligopeptid-Antikörper.

Besonders bevorzugt wird als bindendes Paar Biotin mit Streptavidin bzw. Avidin eingesetzt, so daß der Rezeptor R₁ besonders bevorzugt Biotin enthält.

Die Komponente K des Rezeptors R₁ ist mit der zu bestimmenden Substanz bindefähig. Die Komponente K wird nach der jeweils zu bestimmenden Substanz ausgewählt. Hier sind eine Vielzahl von Rezeptoren geeignet. Zur Bestimmung von Haptenen, Proteinen, DNA oder Zucker ist es besonders vorteilhaft, Antikörper oder sonstige Rezeptoren wie z.B. natürlich vorkommende Bindeproteine, wie Thyroxin bindendes Globulin, gegen diese Substanzen oder deren Fragmente zu verwenden. Besonders bevorzugt wird als Komponente K ein Fab-Fragment verwendet. Zur Bestimmung von Antikörpern ist die Komponente K bevorzugt ein Hapten oder eine Substanz, die ein Epitop, mit dem der Antikörper bindefähig ist, aufweist.

Die Herstellung der Konjugate erfolgt in an sich bekannter Weise (z.B. analog Eur. J. Biochem. 131 (1980) 333-338).

Der zweite für das erfindungsgemäße Verfahren erforderliche Rezeptor R₂ hat mindestens zwei Bindungsstellen für P und weist vorzugsweise eine Vielzahl der zu P komplementären Partner eines spezifisch bindenden Paares auf. Der Rezeptor R₂ vermittelt die Agglutination des sich bei der Reaktion bildenden Komplexes. Da eine Vielzahl dieser anderen Partner des spezifisch bindefähigen Paares im Reaktionssystem vorhanden sind, führt ein natürlich in der Probelösung vorhandener geringer Gehalt von mit diesem Partner bindefähiger Substanz nicht zu Störungen. Der Rezeptor R₂ kann eine Substanz sein, die bereits natürlicherweise mehrere Bindungsstellen für P besitzt, beispielsweise Streptavidin oder Antikörper. Rezeptor R₂ kann polyvalent sein und eine Vielzahl von Bindungsstellen für P aufweisen oder ein Polymer der zu P komplementären Partner des spezifisch bindenden Paares sein, wie z.B. Polystreptavidin. Dabei sind dann die einzelnen Partner miteinander entweder direkt verbunden oder aber über Brücken miteinander verknüpft. Verfahren zur Herstellung derartiger Polymere sind dem Fachmann bekannt und bedürfen keiner näheren Erläuterung.

In einer weiteren Ausführungsform besteht der Rezeptor R₂ aus einem Trägermaterial, an das eine Vielzahl der zu P komplementären spezifisch bindenden Partner gebunden sind. Als Trägermaterialien können Partikel verwendet werden, die üblicherweise eine Größe von 50 bis 1000 nm haben. Geeignete Materialien sind Polystyrol, feinverteiltes Siliziumdioxid, Erythrocyten oder vernetztes Albumin. Die Beschichtung dieser Teilchen mit dem spezifisch bindenden Partner erfolgt nach den dem Fachmann geläufigen Methoden. Verfahren davon sind beispielsweise in EP-PS 73 611 und US-PS 4 703 018 beschrieben.

Die so beschichteten bzw. polymerisierten Rezeptoren R₂ sind universell für das erfindungsgemäße Verfahren einsetzbar und demzufolge nicht parameterspezifisch.

Wesentlich für die Durchführung des erfindungsgemäßen Verfahrens ist es, daß der Rezeptor R₁ nur eine Bindungsstelle für R₂ besitzt, d. h. daß jeder Rezeptor R₁ nur mit einem R₂ reagieren kann. Dies ist eine wesentliche Voraussetzung, da ansonsten R₂ allein durch den Rezeptor R₁ vernetzt werden könnte und damit eine Agglutination bewirkt würde, die nicht auf die nachzuweisende Substanz zurückzuführen ist.

Wenn das erfindungsgemäße Verfahren zur Durchführung von Uptake-Tests verwendet wird, so wird noch ein weiterer Rezeptor R₃ eingesetzt, der mindestens zwei Epitope der zu bestimmenden Substanz aufweist und mit R₁ bindefähig ist. Die Epitope des Rezeptors R₃ entsprechen somit den Epitopen der zu bestimmenden Substanz, die die Bindung an die Komponente K von R₁ bewirken. Bevorzugt wird als Rezeptor R₃ ein Antikörper oder ein Fab₂-Fragment mit Bindestellen für die Komponente K verwendet. Bei der Durchführung des Verfahrens konkurriert dann Rezeptor R₃ mit der Probe um die Bindung an R₁.

In einer weiteren Variante des erfindungsgemäßen Verfahrens wird zusätzlich zu Rezeptor R₃ noch ein Rezeptor R₄ eingesetzt. Dieser Rezeptor R₄ ist dabei die Komponente K des Rezeptors R₁. Bei der Durchführung des Verfahrens konkurrieren dann Rezeptor R₃ und die Probe um die Bindung an Rezeptor R₁ und Rezeptor R₄.

Das Verfahren kann in einer oder mehr Stufen durchgeführt werden. Die Auswertung erfolgt durch Messung des Umfangs der Agglutination. Verfahren hierzu sind bekannt. Geeignet ist beispielsweise die photometrische Trübungsmessung, die Streulichtmessung durch Nephelometrie, Particle Counting oder Photon-Korrelations-Spektroskopie (PCS).

Da jeder der Rezeptoren und auch die zu bestimmende Substanz jeweils nur spezifisch mit dem für ihn bestimmten Reaktionspartner reagieren kann, ist es möglich, alle Rezeptoren und die Probe zusammen zu inkubieren und das Verfahren einstufig durchzuführen. Dies ist besonders vorteilhaft bei der Durchführung des Verfahrens in einem Analysenautomaten.

Die Durchführung aller Verfahrensvarianten erfolgt vorzugsweise in einer gepufferten Lösung. Puffersysteme für diese Verfahren sind an sich bekannt. Besonders geeignet sind hierfür GOOD-Puffer und Phosphatpuffer.

Erfindungsgemäß wird ein Verfahren zur Verfügung gestellt, das einfach und schnell durchzuführen ist und konzentrationsabhängig von der zu bestimmenden Substanz ein Meßsignal liefert. Da das für die immunologische Kompetition zuständige System und das signalbildende System getrennt sind, wird erfindungsgemäß die Empfindlichkeit des Nachweises erhöht. Dadurch können mit einfachen Reagentien sogar niedrigkonzentrierte Substanzen schnell und zuverlässig quantitativ bestimmt werden.

Ein weiterer Gegenstand der vorliegenden Erfindung ist ein Reagenz zur Bestimmung einer spezifisch bindefähigen Substanz, welches dadurch gekennzeichnet ist, daß es Rezeptor R₁, der ein Konjugat aus einem Partner eines spezifisch bindenden Paares P der nur eine Bindungsstelle für R₂ besitzt und einer mit der zu bestimmenden Substanz spezifisch bindefähigen Komponente K ist und Rezeptor R₂, der mindestens zwei Bindungsstellen für P aufweist und in der Probe natürlich nicht vorkommt, enthält.

Das erfindungsgemäße Reagenz kann die einzelnen Rezeptoren R₁ und R₂ und gegebenenfalls R₃ und R₄ vorgemischt oder physikalisch getrennt voneinander enthalten.

Dieses Reagenz ist zur Bestimmung einer Vielzahl von Parametern in Körperflüssigkeiten und Gewebeextrakten geeignet.

In einer bevorzugten Ausführungsform enthält das Reagenz zusätzlich Puffersubstanzen. Besonders bevorzugt enthält es Phosphatpuffer oder GOOD-Puffer.

Die Erfindung wird durch die Figur und die Beispiele erläutert.
- Fig. 1: zeigt ein Schema für zwei Reaktionsprinzipien des erfindungsgemäßen Verfahrens.
- Fig. 2: zeigt ein Schema für zwei Reaktionsprinzipien von Uptake-Tests.

In allen gezeigten Varianten wird als Rezeptor R₂ Streptavidin eingesetzt, das vier Bindungsstellen für P aufweist. Als Rezeptor R₁ wird jeweils ein Konjugat aus einem mit der zu bestimmenden Substanz bindefähigen Rezeptor und Biotin eingesetzt.

Fig. 1a zeigt eine Variante, die zum Nachweis von Antikörpern geeignet ist. Rezeptor R₁ enthält dazu ein mit dem zu bestimmenden Antikörper bindefähiges Epitop.

Variante 1b) dient zur Bestimmung von polyvalenten Substanzen, z.B. Proteinen.

Fig. 2a zeigt eine Variante zur Durchführung eines Uptake-Tests für Thyroxin. Hier wird neben den oben beschriebenen Rezeptoren R₁ und R₂ zusätzlich noch ein Rezeptor R₃ eingesetzt, der ein Anti-T₄-Antikörper ist.

Variante 2b) ist eine weitere Durchführungsform für einen Uptake-Test, bei dem zusätzlich zu den Rezeptoren R₁, R₂ und R₃ noch als Rezeptor R₄ Thyroxin eingesetzt wird.

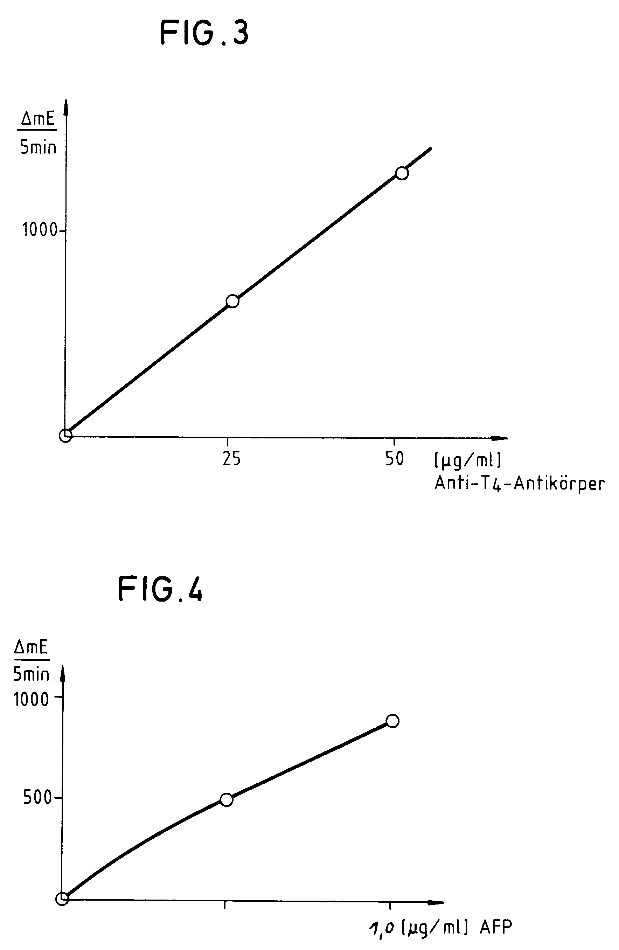

- Fig. 3: zeigt eine Eichkurve für einen Anti-T₄-Antikörpertest
- Fig. 4: zeigt eine Eichkurve für eine AFP-Bestimmung

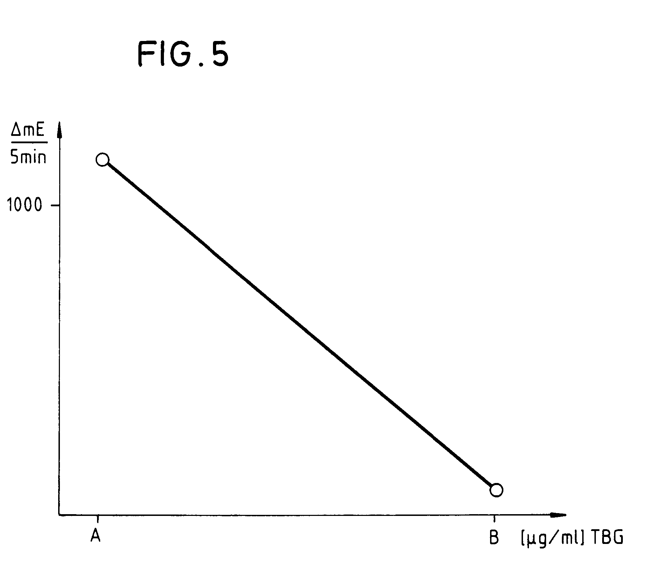

- Fig. 5: zeigt eine Eichkurve für einen T-uptake Test (Prinzip analog Fig. 2a)

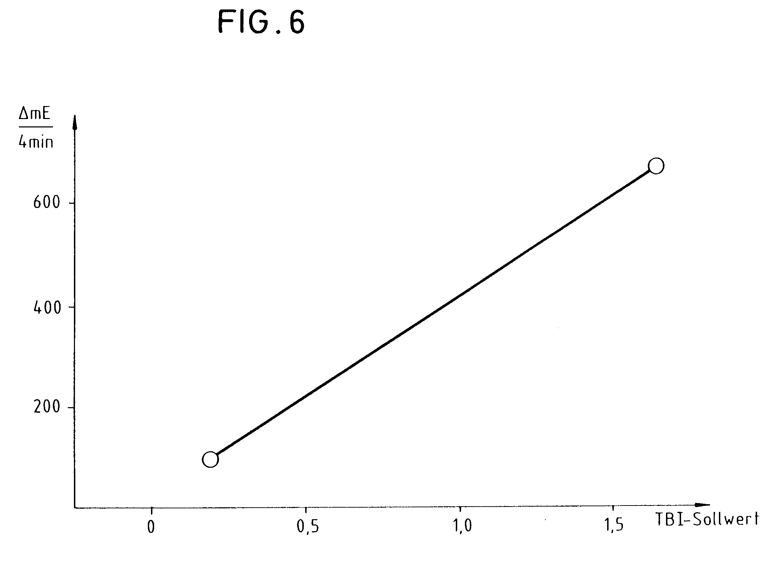

- Fig. 6: zeigt eine Eichkurve für einen T-uptake Test über die Variation der T₄-Menge (Prinzip analog Fig. 2b)

### Beispiel 1

a) Herstellung von Streptavidin-Latex
   Streptavidin in einer Konzentration von 2 mg/ml wird in 15 mMol/l Imidazolpuffer, pH 7,5, 100 mMol/l NaCl zusammen mit Chlormethylstyrolpartikeln (Latex, d = 70 nm, entsprechend USP 4,703,018) in einer Konzentration von 2 Gew.-% 24 Stunden bei 55°C inkubiert und gerührt. Nachdem die Reaktionsmischung während 60 Minuten bei 20 000 Upm zentrifugiert worden ist, wird der Überstand abdekantiert und der Niederschlag in 200 mMol/l Glycin-Puffer, pH 7,5, mit 0,5 % Rinderserumalbumin aufgenommen. Durch entsprechende Verdünnung wird ein 1 Gew.-%iges Streptavidin-Latexreagenz hergestellt.
b) Herstellung von Hapten-Biotin-Konjugaten
   Hierzu wird n-Butyloxycarbonyl-tetrajodothyronin
   (DE-A 28 05 961) über Pentamethylendiamin mit Biotin gekoppelt, wie in Eur. J. Biochem. 131 (1980) 333-338 beschrieben. Man erhält T₄-Biotin-Konjugat.

### Beispiel 2

### Bestimmung eines Antikörpers gegen T₄

### Reagenz 1:

0,1 µmol/l T₄-Biotin-Konjugat
0,1 mol/l Natriumbarbiturat-Puffer pH 8,5
2 Gew.-% Dextran

### Reagenz 2:

10 mg/ml Streptavidin-Latex
200 mmol/l Glycin-Puffer pH 7,5
0,1 Gew.-% Natriumazid

Als Probe wird ein polyklonaler Antikörper gegen T₄ in physiologischer Kochsalzlösung, unter Zusatz von 0,1 % unspezifischem Schaf-Immunglobulin (IgG), verwendet.

### Durchführung der Bestimmung:

20 µl Probe und 960 µl Reagenz 1 werden für fünf Minuten bei 37°C inkubiert. Danach wird die Agglutinationsreaktion durch Zugabe von 20 µl Reagenz 2 gestartet und am Photometer bei 405 nm die Änderung der optischen Dichte pro Zeiteinheit gemessen. Das Ergebnis zeigt Fig. 3.

### Beispiel 3

### Bestimmung von AFP ( α-Foetoprotein)

a) Herstellung eines Konjugats aus Biotin und Fab-Fragmenten von Anti-AFP-Antikörpern (Anti-AFP-Fab-Biotin)
   Polyklonaler Antikörper gegen AFP wird immunsorptiv aufgereinigt und nach Analyt. Biochem. 161 (1987) 262-271 bzw. Analyt. Biochem. 149 (1985) 529-536 an Biotin gekoppelt.
b) Testdurchführung
   Reagenz 1
      5 µg/l Anti-AFP-Fab-Biotin
      0,1 mol/l Natriumbarbiturat-Puffer pH 8,5
   Reagenz 2
      10 mg/ml Streptavidin-Latex
      0,2 mol/l Glycin-Puffer pH 7,5
      0,1 Gew.-% Natriumazid

   Als Probe wird AFP in Humanserum verwendet.
   50 µl Probe und 900 µl Reagenz 1 werden bei 37°C für fünf Minuten inkubiert. Danach wird die Agglutinationsreaktion durch Zugabe von 20 µl Reagenz 2 gestartet und die Änderung der optischen Dichte pro Zeiteinheit am Photometer bei 405 nm gemessen. Das Ergebnis zeigt Figur 4.

### Beispiel 4

### T-uptake-Test (Reaktionsprinzip gemäß Fig. 2a)

Das Testprinzip besteht darin, daß ein Anti-T₄-Antikörper und ein T₄-Biotin-Konjugat um das TBG (Thyroxinbindendes Globulin) der Probe konkurrieren.

### Durchführung der Bestimmung

Reagenz 1:
   40 nmol/l T₄-Biotin-Konjugat
   0,1 mol/l Natriumphosphat-Puffer pH 7,5
   1 Gew.-% Dextransulfat
Reagenz 2:
   0,1 mg/ml polyklonaler Anti-T₄-Antikörper aus Schaf (IgG)
   10 mg/ml Streptavidin-Latex
   200 mmol/l Glycin-Puffer pH 7,5
   0,1 Gew.-% Natriumazid

Als Probe wird verwendet physiologische Kochsalzslösung (A) und 100 µg/ml TBG in physiologischer Kochsalzlösung (B).

20 µl Probe und 960 µl Reagenz 1 werden bei 37°C für fünf Minuten inkubiert. Danach wird die Agglutinationsreaktion durch Zugabe von 20 µl Reagenz 2 gestartet und am Photometer die Änderung der optischen Dichte pro Zeiteinheit bei 405 nm gemessen. Das Ergebnis zeigt Fig. 5.

### Beispiel 5

### T-uptake-Test (Reaktionsprinzip gemäß Fig. 2b)

Die Bestimmung erfolgt in der Weise, daß T₄ zur Probe zugegeben wird, um überschüssiges TBG abzusättigen. Danach wird das nicht gebundene T₄ gemessen. Damit wird eine dem Thyroxinbindungsindex (TBI) direkt proportionale Eichkurve erhalten.
Reagenz 1:
   1 µg/ml T₄
   0,1 mol/l Natriumphosphat-Puffer pH 7,5
Reagenz 2:
   0,1 mol/l Natriumbarbiturat-Puffer pH 8,5
   2 Gew.-% Dextransulfat
   0,2 mg/ml Streptavidin-Latex
   2 µg/ml polyklonaler Anti-T₄-Antikörper aus Schaf (IgG)
Reagenz 3:
   2 µmol/l T₄-Biotin-Konjugat in Ethanol/Wasser (1 : 1)

Als Probe wird Humanserum verwendet, welches definierte Thyroxinbindungsindex (TBI)-Sollwerte besitzt (Probe A: 0,19 TBI, Probe B: 1,64 TBI)

### Durchführung der Bestimmung:

20 µl Probe, 20 µl Reagenz 1 und 900 µl Reagenz 2 werden bei 37°C für fünf Minuten inkubiert. Danach wird die Agglutinationsreaktion durch Zugabe von 20 µl Reagenz 3 gestartet und die Änderung der optischen Dichte pro Zeiteinheit am Photometer bei 405 nm gemessen. Das Ergebnis zeigt Fig. 6.

## Patentansprüche

1. Verfahren zur Bestimmung einer spezifisch bindefähigen Substanz durch Inkubation der Probelösung mit mindestens zwei Rezeptoren R₁ und R₂, wobei R₁ und R₂ miteinander bindefähig sind und R1 mit der zu bestimmenden Substanz spezifisch bindefähig ist und Messung der bei der Reaktion auftretenden Agglutination,
**dadurch gekennzeichnet**,
daß als Rezeptor R₁ ein Konjugat aus einem Partner eines spezifisch bindenden Paares P, der nur eine Bindungsstelle für R₂ besitzt und einer mit der zu bestimmenden Substanz spezifisch bindefähigen Komponente K und als R₂ ein Rezeptor, der mindestens zwei Bindungsstellen für P aufweist und in der Probelösung nicht schon natürlich vorhanden ist, verwendet wird.

2. Verfahren nach Anspruch 1,
**dadurch gekennzeichnet**,
daß eine Substanz bestimmt wird, die mindestens zwei mit R₁ bindefähige Epitope aufweist.

3. Verfahren nach Anspruch 1 oder 2,
**dadurch gekennzeichnet**,
daß als Rezeptor R₂ ein Rezeptor verwendet wird, der eine Vielzahl der zu P komplementären Partner des spezifisch bindenden Paares aufweist.

4. Verfahren nach einem der vorhergehenden Ansprüche,
**dadurch gekennzeichnet**,
daß als Rezeptor R₁ ein Konjugat aus einem Partner P eines spezifisch bindenden Paares und einem Fab-Fragment eines für die zu bestimmende Substanz spezifischen Antikörpers als Komponente K verwendet wird.

5. Verfahren nach einem der vorhergehenden Ansprüche,
**dadurch gekennzeichnet**,
daß zusätzlich ein Rezeptor R₃ verwendet wird, der mindestens zwei Epitope der zu bestimmenden Substanz aufweist und mit R₁ bindefähig ist.

6. Verfahren nach Anspruch 5,
**dadurch gekennzeichnet**,
daß als Rezeptor R₃ ein Antikörper oder dessen Fab₂-Fragment verwendet wird, der mit der Komponente K des Rezeptors R₁ bindefähig ist.

7. Verfahren nach einem der vorhergehenden Ansprüche,
**dadurch gekennzeichnet**,
daß zusätzlich ein Rezeptor R₄ eingesetzt wird, der der Komponente K des Rezeptors R₁ entspricht.

8. Reagenz zur Bestimmung einer spezifisch bindefähigen Substanz nach einem der Ansprüche 1 bis 7,
**dadurch gekennzeichnet**,
daß es Rezeptor R₁, der ein Konjugat aus einem Partner eines spezifisch bindenden Paares P, der nur eine Bindungsstelle für R₂ besitzt und einer mit der zu bestimmenden Substanz spezifisch bindefähigen Komponente K ist und Rezeptor R₂, der mindestens zwei Bindungsstellen für P aufweist und in der Probe natürlich nicht vorkommt, enthält.

9. Reagenz nach Anspruch 8,
**dadurch gekennzeichnet**,
daß es zusätzlich einen Rezeptor R₃, der mindestens zwei Bindungsstellen, die mit der Komponente K bindefähig sind und einem Epitop der zu bestimmenden Substanz entsprechen, enthält.

10. Reagenz nach einem der Ansprüche 8 und 9,
**dadurch gekennzeichnet**,
daß es zusätzlich einen Rezeptor R₄ enthält, der der Komponente K entspricht.

## Claims

1. Process for the determination of a specifically bindable substance by incubation of the sample solution with at least two receptors R₁ and R₂, whereby R₁ and R₂ are bindable with one another and R₁ is specifically bindable with the substance to be determined and measurement of the agglutination occurring in the case of the reaction, characterised in that, as receptor R₁, a conjugate is used of a partner of a specifically binding pair P which possesses only one binding position for R₂ and of component K specifically bindable with the substance to be determined and, as R₂, a receptor which has at least two binding positions for P and is not already naturally present in the sample solution.

2. Process according to claim 1, characterised in that a substance is determined which has at least two epitopes bindable with R₁.

3. Process according to claim 1 or 2, characterised in that, as receptor R₂, a receptor is used which has a plurality of the partner of the specifically binding pair complementary to P.

4. Process according to one of the preceding claims, characterised in that, as receptor R₁, there is used a conjugate of a partner P of a specifically binding pair and a Fab fragment of an antibody specific for the substance to be determined as component K.

5. Process according to one of the preceding claims, characterised in that there is addionally used a receptor R₃ which has at least two epitopes of the substance to be determined and is bindable with R₁.

6. Process according to claim 5, characterised in that, as receptor R₃, an antibody or its Fab₂ fragment is used which is bindable with the component K of the receptor R₁.

7. Process according to one of the preceding claims, characterised in that there is additionally used a receptor R₄ which corresponds to the component K of the receptor R₁.

8. Reagent for the determination of a specifically bindable substance according to one of claims 1 to 7, characterised in that it contains receptor R₁, which is a conjugate of a partner of a specifically binding pair P which only possesses one binding position for R₂ and of a component K specifically bindable with the substance to be determined, and receptor R₂ which has at least two binding positions for P and does not occur naturally in the sample.

9. Reagent according to claim 8, characterised in that it additionally contains a receptor R₃ which has at least two binding positions which are bindable with the component K and correspond to an epitope of the substance to be determined.

10. Reagent according to one of claims 8 and 9, characterised in that it additionally contains a receptor R₄ which corresponds to the component K.

## Revendications

1. Procédé de détermination d'une substance capable de se lier spécifiquement par incubation de la solution échantillon avec au moins deux récepteurs R₁ et R₂, R₁ et R2 étant capables de se lier l'un à l'autre et R₁ étant capable de se lier spécifiquement à la substance à déterminer, et mesure de l'agglutination qui se produit lors de la réaction, caractérisé en ce que l'on utilise comme récepteur R₁ un conjugué constitué par un partenaire P d'une paire qui se lie spécifiquement, qui ne comporte qu'un site de liaison pour R₂, et par un composant K capable de se lier spécifiquement à la substance à déterminer et comme R₂ un récepteur qui comporte au moins deux sites de liaison pour P et qui n'est pas déjà présent naturellement dans la solution échantillon.

2. Procédé selon la revendication 1, caractérisé en ce que l'on détermine une substance qui comporte au moins deux épitopes capables de se lier à R₁.

3. Procédé selon la revendication 1 ou 2, caractérisé en ce que l'on utilise comme récepteur R₂ un récepteur qui comporte une multiplicité de partenaires, complémentaires de P, de la paire qui se lie spécifiquement.

4. Procédé selon l'une des revendications précédentes, caractérisé en ce que l'on utilise comme récepteur R₁ un conjugué constitué par un partenaire P d'une paire qui se lie spécifiquement et par un fragment Fab d'un anticorps spécifique de la substance à déterminer comme composant K.

5. Procédé selon l'une des revendications précédentes, caractérisé en ce que l'on utilise en outre un récepteur R₃ qui comporte au moins deux épitopes de la substance à déterminer et qui est capable de se lier à R₁.

6. Procédé selon la revendication 5, caractérisé en ce que l'on utilise comme récepteur R₃ un anticorps, ou son fragment Fab₂, qui est capable de se lier au composant K du récepteur R₁.

7. Procédé selon l'une des revendications précédentes, caractérisé en ce que l'on utilise en outre un récepteur R₄ qui correspond au composant K du récepteur R₁.

8. Réactif pour la détermination d'une substance capable de se lier spécifiquement selon l'une des revendications 1 à 7, caractérisé en ce qu'il contient un récepteur R₁, qui est un conjugué constitué par un partenaire P d'une paire qui se lie spécifiquement, qui ne possède qu'un site de liaison pour R₂, et par un composant K capable de se lier spécifiquement à la substance à déterminer, et un récepteur R₂, qui comporte au moins deux sites de liaison pour P et qui n'est pas présent naturellement dans l'échantillon.

9. Réactif selon la revendication 8, caractérisé en ce qu'il contient en outre un récepteur R₃ qui présente au moins deux sites de liaison qui sont capables de se lier au composant K et qui correspondent à un épitope de la substance à déterminer.

10. Réactif selon l'une des revendications 8 et 9, caractérisé en ce qu'il contient en outre un récepteur R₄ qui correspond au composant K.
